# EUROPEAN PATENT APPLICATION

(11) **EP 4 394 663 A1**
(43) Date of publication of application: **03.07.2024**
(21) Application number: 23217869.9
(22) Date of filing: 19.12.2023
(51) Int. Cl.: G06N 3/088, G06N 3/0895, G06N 3/09, G06N 5/01, G06N 20/10, G06N 20/20, G01N 33/18

(54) **PREDICTION OF WATER PARAMETERS BASED ON MULTIVARIATE REGRESSION TREES ESTIMATION**

(30) Priority: 19.12.2022 IL 29928122
(71) Applicant: Maytronics Ltd., 1935000 Kibbutz Yizrael (IL)
(72) Inventor: AMIT, Bar, 3881200 Talmi Elazar (IL); DUVDEVANI, Adva, 1923200 HaYogev (IL); GUPTA, Vishal, 201204 Modinagar (IN)
(74) Representative: J A Kemp LLP

(57) **Abstract**

A method of estimating a level of one or more water parameters using trained machine learning (ML) based regression trees, comprising receiving physical features and spectral features relating to one or more samples of water, propagating the water sample(s) through a regression tree comprising a plurality of nodes arranged in a plurality of branches propagating from a root node to a plurality of leaf nodes, each of the plurality of nodes is associated with an ML model trained to estimate a level of one or more water parameters in water samples, identifying a certain leaf node to which the water sample(s) propagated based on the plurality of physical features, estimating a level of one or more of the water parameters in the water sample(s) by applying the ML model associated with the certain leaf node to the spectral features, and initiating action(s) based on the water parameter(s) estimated level

## Description

### BACKGROUND

The present invention, in some embodiments thereof, relates to estimating water parameters in water samples, and, more specifically, but not exclusively, to estimating water parameters in water samples using trained multivariate regression trees.

Ensuring high water quality, cleanliness and purity in artificial water bodies such as, for example, swimming pools, reservoirs, fountains, and/or the like may be highly desired and typically essential for safety, health and/or hygiene related concerns.

One of the most common practices for treating and purifying water in such artificial water bodies comprises monitoring water parameters, for example, chlorine concentration, oxygen, acidity (pH), alkalinity and/or the like and taking one or more actions accordingly, for example, adding to the water chlorine or one or more related compounds, for example, chloramine, chlorine dioxide, and/or the like which are strong agents capable of rapidly killing many harmful micro-organisms.

### SUMMARY

According to a first aspect of the present invention there is provided a method of creating machine learning (ML) based regression trees trained for estimating a level of one or more water parameters, comprising using one or more processors for using a plurality of training samples relating to a plurality of water samples to create a regression tree comprising a plurality of nodes arranged in a plurality of branches extending from a root node to a plurality of leaf nodes and outputting the regression tree for estimating the level of the one or more water parameter in one or more new water samples. The regression tree is created by splitting each of the plurality of nodes into at least two respective child nodes by:
- Splitting a set of the plurality of training samples which propagated to the respective node into at least two subsets based on each of a plurality of candidate split values applied for each of one or more of a plurality of physical features of the propagated samples.
- For each of the candidate splits, training two ML models each associated with one of the child nodes. Each ML model is trained using a plurality of spectral features or an ORP value of the propagated samples of a respective one of the two subsets and its loss is computed.
- Selecting an optimal split value from of the plurality of candidate split values by minimizing loss of each of the trained ML models applied to estimate a level of one or more water parameters in the propagated samples of the respective subset or not splitting if neither split improves the loss.
And outputting the regression tree for estimating the level of the one or more water parameter in one or more new water samples.

According to a second aspect of the present invention there is provided a system for creating machine learning (ML) based regression trees trained for estimating a level of one or more water parameters, comprising one or more processors configured to execute a code. The code comprising:
- Code instructions to use a plurality of training samples relating to a plurality of water samples to create a regression tree comprising a plurality of nodes arranged in a plurality of branches extending from a root node to a plurality of leaf nodes by splitting each of the plurality of nodes into at least two respective child nodes by, splitting a set of the plurality of training samples which propagated to the respective node into at least two subsets based on each of a plurality of candidate split values applied for each of one or more of a plurality of physical features of a plurality of the training samples, training two ML models each associated with one of the child nodes, each ML model is trained using a plurality of spectral features or an ORP value of the propagated samples of a respective one of the two subsets, and selecting an optimal split value from of the plurality of candidate split values by minimizing loss of each of the trained ML models applied to estimate a level of one or more water parameters in the propagated samples of the respective subset.Code instructions to output the regression tree for estimating the level of the one or more water parameters in one or more new water samples.

According to a third aspect of the present invention there is provided a method of estimating a level of one or more water parameters using trained machine learning (ML) based regression trees, comprising using one or more processors for:
- Receiving a plurality of physical features and a plurality of spectral features relating to one or more samples of water.
- Propagating the one or more samples of water through a regression tree comprising a plurality of nodes arranged in a plurality of branches propagating from a root node to a plurality of leaf nodes. Each of the plurality of nodes is associated with an ML model trained to estimate a level of one or more water parameters in water samples.
- Identifying a certain leaf node of the plurality of leaf nodes to which the one or more water samples propagated based on the plurality of physical features.
- Estimating a level of the one or more water parameters in the one or more water samples by applying the ML model associated with the certain leaf node to the plurality of spectral features or the ORP level.
- Initiating one or more actions based on the estimated level of the one or more water parameters.

According to a fourth aspect of the present invention there is provided a system for estimating a level of one or more water parameters using trained machine learning (ML) based regression trees, comprising one or more processors configured to execute a code. The code comprising:
- Code instructions to receive a plurality of physical features and a plurality of spectral features or ORP level relating to one or more samples of water.
- Code instructions to propagate one or more samples of water through a regression tree comprising a plurality of nodes arranged in a plurality of branches propagating from a root node to a plurality of leaf nodes. Each of the plurality of nodes is associated with an ML model trained to estimate the level of one or more water parameters in water samples.
- Code instructions to identify a certain leaf node of the plurality of leaf nodes to which the one or more water samples propagated based on the plurality of physical features.
- Code instructions to estimate a level of the one or more water parameters in the one or more water samples by applying the ML model associated with the certain leaf node to the ORP or the plurality of spectral features.
- Code instructions to initiate one or more actions based on the estimated level of the one or more water parameters.

In a further implementation form of the first, second, third and/or fourth aspects, the one or more water parameter are members of a group consisting of: a chlorine concentration value, a dissolved oxygen concentration value, an alkalinity value, Oxidation reduction potential value and/or an acidity value.

In a further implementation form of the first, second, third and/or fourth aspects, the ML models are linear ML models.

In a further implementation form of the first, second, third and/or fourth aspects, the plurality of physical features are members of a group consisting of: temperature, pH, electrical conductivity, acidity, basicity, cyanuric acid, alkalinity, and/or hardness.

In a further implementation form of the first, second, third and/or fourth aspects, the plurality of spectral features comprise light absorption value in each of a plurality of light spectral regions or ORP value.

In a further implementation form of the first, second, third and/or fourth aspects, one or more of the plurality of physical features are expressed in a continuous range.

In a further implementation form of the first, second, third and/or fourth aspects, one or more of the plurality of physical features are expressed in discrete values.

In a further implementation form of the first and/or second aspects, each of the plurality of training samples is associated with a respective value of a level of the one or more water parameters.

In a further implementation form of the first and/or second aspects, the loss is computed based on the level of the one or more water parameters of the plurality of training samples.

In a further implementation form of the first and/or second aspects, the plurality of candidate split values are selected to ensure a minimal predefined number of samples is propagated to each of the child nodes.

In a further implementation form of the first and/or second aspects, in case a minimized loss computed for a certain node does not exceed the loss computed for its parent node, the parent node is not split and is defined as a leaf node.

In a further implementation form of the first and/or second aspects, one or more of the candidate split values are selected based on a plurality of quantiles segmenting the continuous range of one or more of the physical features.

In a further implementation form of the first and/or second aspects, each of the ML models associated with one of the at least two child nodes is trained using a first group of training samples selected from one of the subsets which is propagated to the respective child node and tested using a second group of validation samples of the respective subset such that the first group is not overlapping with the second group.

In a further implementation form of the first and/or second aspects, the first group is larger than the second group.

In a further implementation form of the first and/or second aspects, the plurality of samples are samples of water of one or more pools.

In an optional implementation form of the first and/or second aspects, a forest of regression trees comprising a plurality of regression trees is created. Each of the plurality of regression trees is created based on a different association of its plurality of nodes with respective ML models.

In an optional implementation form of the third and/or fourth aspects, the level of one or more of the water parameters in one or more of the water samples is estimated using a forest of regression trees comprising a plurality of regression trees, each of the plurality of regression trees is configured with different association of its plurality of nodes with respective ML models.

Other systems, methods, features, and advantages of the present disclosure will be or become apparent to one with skill in the art upon examination of the following drawings and detailed description. It is intended that all such additional systems, methods, features, and advantages be included within this description, be within the scope of the present disclosure, and be protected by the accompanying claims.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks automatically. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of methods and/or systems as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars are shown by way of example and for purposes of illustrative discussion of embodiments of the invention. In this regard, the description taken with the drawings makes apparent to those skilled in the art how embodiments of the invention may be practiced.

In the drawings:
FIG. 1A and FIG. 1B present a flowchart of an exemplary process of training one or more regression trees to estimate level (value) of one or more water parameters in water samples, according to some embodiments of the present invention;
FIG. 2 is a schematic illustration of an exemplary system for training one or more regression trees to estimate level (value) of one or more water parameters in water samples, according to some embodiments of the present invention;
FIG. 3 is a schematic illustration of an exemplary regression tree trained for estimating a level (value) of one or more water parameters in water samples, according to some embodiments of the present invention;
FIG. 4 is a flowchart of an exemplary process of estimating a level (value) of one or more water parameters in water samples using one or more trained regression trees, according to some embodiments of the present invention; and
FIG. 5 is a schematic illustration of an exemplary system for estimating a level (value) of one or more water parameters in water samples using one or more trained regression trees, according to some embodiments of the present invention.

### DETAILED DESCRIPTION

The present invention, in some embodiments thereof, relates to estimating water parameters in water samples, and, more specifically, but not exclusively, to estimating water parameters in water samples using multivariate regression trees.

Maintaining effective and accurate levels of one or more water parameters, for example, concentration of (free) Chlorine one or more of its compounds and/or one or more other water parameters such as, for example, dissolved oxygen concentration, alkalinity, acidity (pH) and/or the like in bodies of water, whether natural (e.g., pools, springs, wells, ponds, etc.) and/or artificial (e.g., swimming pools, spas, reservoirs, fountains, etc.) may be essential to ensure water quality, purity and/or cleanliness.

However, measuring the water parameter(s), for example, concentration of (free) chlorine in water samples of such bodies of water using chemical and/or electrochemical test equipment, procedures and/or methods may be highly complex, costly and time consuming since the chemical test equipment may be highly expensive, inaccessible and/or require expertise such that in order to measure the water parameter(s), the water samples may need to be sent to expert laboratories equipped with such equipment. Moreover, these existing chemical and electrochemical test equipment may require maintenance and calibration which may significantly increase complexity and/or cost of the water parameters estimation. Furthermore, the existing chemical and electrochemical test equipment may degrade over time which may lead to inaccurate readings and/or estimation of the water parameters.

According to some embodiments of the present invention, there are provided methods, systems and computer program products for estimating (predicting) concentration of chlorine, dissolved oxygen concentration, alkalinity, acidity (pH) and/or the like in water samples taken from one or more natural and/or artificial bodies of water, designated water bodies herein after. In particular, levels of the water parameters may be estimated using one or more trained regression trees, specifically multivariate regression trees, for example, a Partial Least Squares (PLS) tree, Symbolic regression trees with parametric equation using curve fit, an Orthogonal PLS (OPLS) tree, a Two-way Orthogonal PLS (O2PLS) tree, and/or the like.

Such multivariate regression trees may be configured, as known in the art, to apply linear regression (linear models) or multivariate log-linear regression for the prediction rather than use fixed values, as may be done for regular regression trees, and thus learn and/or identify relations between multiple variants. As such, the nodes of the regression tree may be associated with a Machine Learning (ML) model, for example, a linear model, a neural network, a classifier, a Support Vector Machine (SVM), Symbolic regression with estimated weights and/or the like which may be trained and used to estimate a level of one or more of the water parameter(s), for example, chlorine concentration in data samples which propagated to the respective node.

The regression tree(s) may be trained to estimate (predict) levels of one or more of the water parameters in water samples based on physical features and spectral features or an ORP value of the water sample which may be captured, extracted and/or measured using simple, low-cost and accessible test equipment which may be deployed in and/or next to the water bodies, for example, a water treatment system, a water cleaning and/or purifying system, a pool robot and/or the like. Optionally and even typically such systems deployed at the water bodies may be automated systems configured to automatically collect the physical features and the spectral features from samples of water of the body of water.

The physical features, for example, temperature, electrical conductivity, acidity, basicity, cyanuric acid, alkalinity, hardness, and/or the like may be extracted and/or measured for the water samples using simple low cost and highly accessible chemical, electrochemical and/or other physical test equipment. The spectral features, for example, values of light absorption (response) of the water samples in one or more light spectral regions, for example, Ultra-Violet (UV), fluorescence UV, UV visible, Infrared (IR), Near Infrared (NIR), fluorescence NIR, and/or the like may be captured and/or measured using simple, low cost and accessible spectral test equipment, for example, a spectroscopic device, a spectrometer, and/or the like configured to apply one or more spectroscopic techniques as known in the art for measuring the light absorption of the water samples.

The regression tree(s) may be constructed and trained using a plurality of training samples relating to a plurality of water samples. Each training sample may comprise one or more physical features and one or more spectral features captured, measured, and/or extracted from a respective water sample. One or more of the training samples may be further associated with a level and/or value of one or more of the water parameters in the respective water sample. For example, the training samples may be labeled with labels indicative of the levels (values) of one or more of the water parameters measured for the water samples.

The regression tree may be created by propagating the training samples through the nodes of the tree in an iterative process starting from a root node and extending through a plurality of branches to end leaf nodes which are not further split. During the training, the nodes of the regression tree may be split according to optimal split values which yield best performance, for example, accuracy, reliability, consistency and/or the like in prediction of the level of the water parameter(s) in the training samples by the ML models associated with the nodes. The iterative process may be repeated until reaching nodes which when split do not improve prediction performance and are therefore set as leaf nodes.

In particular, the nodes may be split according to split values which may be based only on the physical features of the training samples while the ML models associated with the nodes may be trained to estimate the level of the water parameter(s) based only on the spectral features, such that there is a separation between the split values selected for the nodes and the data used for estimating the level of the water parameter(s) at the nodes.

During the training a plurality of candidate split values may be evaluated for each node of the regression tree. The training samples which propagated to the respective node may be split into two subsets according to each candidate split value, the two subsets may be propagated down to two or more child nodes of the respective node and the ML models associated with the child nodes may be trained using the respective subsets propagated to their associated nodes.

One or more of the physical features may be expressed in continuous ranges and/or scales. In such case, one or more candidate split values may be optionally selected for one or more of the nodes based on quantiles segmenting the continuous range and/or scale of one or more of the physical features rather than defining singular candidate split values. In other words, rather than selecting a plurality of single values as candidate split values, quantiles may be selected as candidate split values for one or more of the continuous range physical features.

The prediction performance of the ML models may be evaluated by comparing between the values of level of the water parameter(s) in the training samples predicted by the trained ML models and the actual level (value) of the water parameter(s) associated with the training samples. For each node, the split value which yields best performance of the ML models may be selected as the split value for the respective node. In case performance is not improved by the ML models associated with the child nodes compared to the performance of the ML model associated with the respective (parent) node, the node may not be further split and may be defined as a leaf node.

Optionally, the training samples propagated to each node may be divided into a plurality of non-overlapping groups, for example, a first group comprising training samples used to train the ML model associated with the respective node, a second group comprising training samples used to test the ML model, and a third group comprising training samples used to verify the ML model.

Optionally, a plurality of regression trees are constructed and trained to form a forest of regression trees where each tree may be constructed differently, for example, using different ML models associated with their nodes.

The trained regression tree(s) may be then applied and used for estimating the level of one or more of the water parameters, for example, chlorine concentration in one or more new (previously unseen) water samples based on one or more physical features and one or more spectral features extracted from the respective water sample. In particular, each water sample may be propagated through the trained regression tree(s) according to its physical features until reaching a leaf node where the ML model associated with the leaf node may be applied to estimate the level of one or more of the water parameters in the respective water based on its spectral features or its ORP based equation (with other water parameters).

As seen, there may be some overlap between the physical features measured in the water sample(s) and the water parameters predicted accordingly using the regression tree(s). This is because in some embodiments, one or more of the water parameters may be predicted while in other embodiments the same water parameter(s)_may be measured and used for predicting one or more of the other water parameters. For example, in some embodiments, an alkalinity (value) of the water may be predicted based on one or more physical features measured in one or more water samples, for example, acidity, temperature, electrical conductivity, and/or the like. However, in other embodiments, the alkalinity (value) of the water may be measured and used to predict another water parameter, for example, chlorine concentration.

Using trained regression trees for estimating levels (values) of one or more of the water parameters in water samples may present major advantages and benefits compared to existing water quality measurement systems and methods.

First, measuring levels of the water parameters in water samples as may be done by the existing methods may require expensive, complex and/or inaccessible chemical and/or electrochemical test equipment which may optionally require expertise and/or experience to operate. Moreover, such chemical and/or electrochemical test equipment may require frequent maintenance and/or calibration which may significantly increase complexity and/or cost of its use. In addition, such chemical and electrochemical test equipment may degrade over time which may result in inaccurate readings and/or estimation of the water parameters' levels. Furthermore, due to the complexity and/or inaccessibility of the test equipment, in order to measure the water parameter(s), for example, chlorine concentration in the water samples, the water samples may need to be sent to remote laboratories equipped with such test equipment.

In contrast, using the trained regression trees to estimate the values of the water parameter(s) in water samples may overcome these limitations since the regression trees are trained to estimate the water parameter(s) based on physical features and spectral features or an ORP value which may be easily measured and/or captured for the water samples using simple, low-cost, and highly accessible test equipment. Moreover, such simple, low-cost, and highly accessible test equipment may be optionally deployed in and/or next to the water bodies such that the water parameter(s) estimation may be done frequently and possibly continuously thus ensuring constant, effective and accurate monitoring of the water parameter(s) of the water.

Moreover, configuring and/or defining the split values of the nodes of the regression tree based on quantiles segmenting continuous ranges of the physical values may significantly reduce complexity and/or size of the regression tree or the logarithmic parametric model since using segments of the range rather than singular values in the range may significantly reduce the number of split options and thus the number of nodes. As result, the number of ML models associated with the nodes which are trained and used may be also significantly reduced. The computing resources, for example, processing resources, storage resources, network resources, processing time and/or the like required for training the ML models may be therefore reduced. Constructing the regression tree based on quantiles of the continuous range physical feature(s) may be of particular benefit for large datasets of training data (samples) since evaluating each value of these features as may be done by traditional methods may significantly increase the computing resources utilized for the training process.

Furthermore, creating a forest of regression tress configured differently and applying the forest to estimate the water parameter(s) in the water samples may significantly increase accuracy, reliability and/or consistency of the estimation (prediction). Since the estimation is averaged between multiple prediction models the contribution of each single regression tree is weighted down thus reducing the impact of potential prediction errors and/or inaccuracies of any single one or more regression tree models on the overall estimation outcome.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

As will be appreciated by one skilled in the art, aspects of the present invention may be embodied as a system, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer readable program code embodied thereon.

Any combination of one or more computer readable medium(s) may be utilized. The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer program code comprising computer readable program instructions embodied on a computer readable medium may be transmitted using any appropriate medium, including but not limited to wireless, wire line, optical fiber cable, RF, etc., or any suitable combination of the foregoing.

The computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

The computer readable program instructions for carrying out operations of the present invention may be written in any combination of one or more programming languages, such as, for example, assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages.

The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown 14uccesssion may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

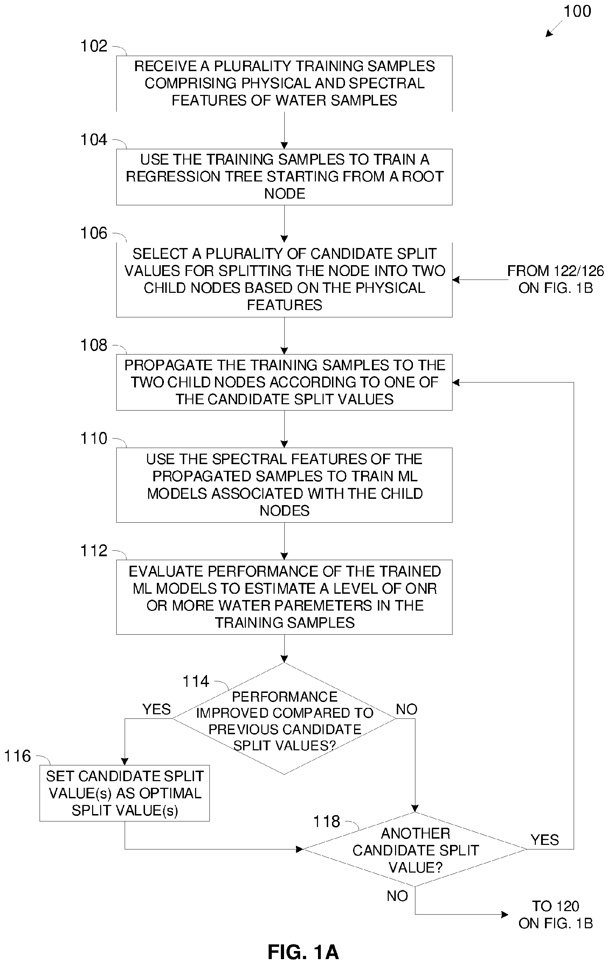

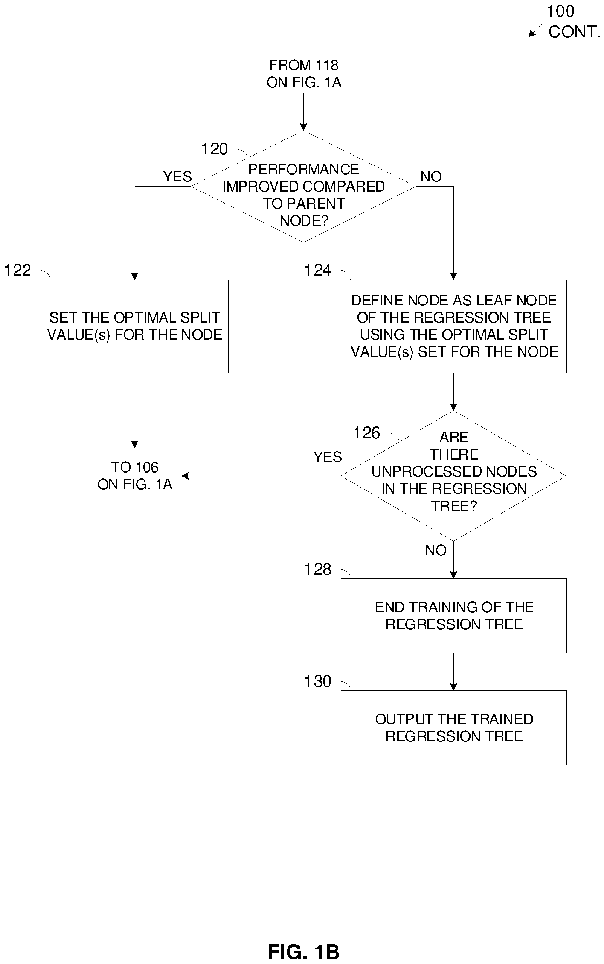

Referring now to the drawings, FIG. 1A and FIG. 1B present a flowchart of an exemplary process of training one or more regression trees to estimate a level (value) of one or more water parameters in water samples, according to some embodiments of the present invention.

An exemplary process 100 may be executed to estimate a level (value) of one or more water parameters of one or more bodies of water, for example, concentration of (free) chlorine, dissolved oxygen concentration, alkalinity, acidity (pH), and/or the like impacting spectral features of the water.

Specifically, rather than applying complex and/or costly chemical tests, the level of the water parameter(s) is estimated using one or more trained regression trees applied to one or more physical features (e.g., chemical features, electrical features, geographical features, etc.) and one or more spectral features measured in water samples of the body(s) of water.

The regression tree(s) are trained using a plurality of training samples relating to a plurality of training water samples. The training samples may each comprise one or more physical features (and one or more spectral features of the training water samples. In particular, each regression tree is created by propagating the training samples through nodes of the tree which are, if this improves the performance, split according to optimal splits of the physical features of the samples where the optimal splits are identified based on the level of the water parameter(s) prediction made by ML models associated with the nodes.

The trained regression tree may be then applied and used to estimate the level of the water parameter(s) in one or more new and previously unseen water samples based on one or more physical features and one or more spectral features extracted from the respective water sample.

As stated herein before, one or more of the water parameters which may be predicted using the regression tree(s) may be optionally measured using test and/or measurement used as physical features for predicting one or more of the other water parameters.

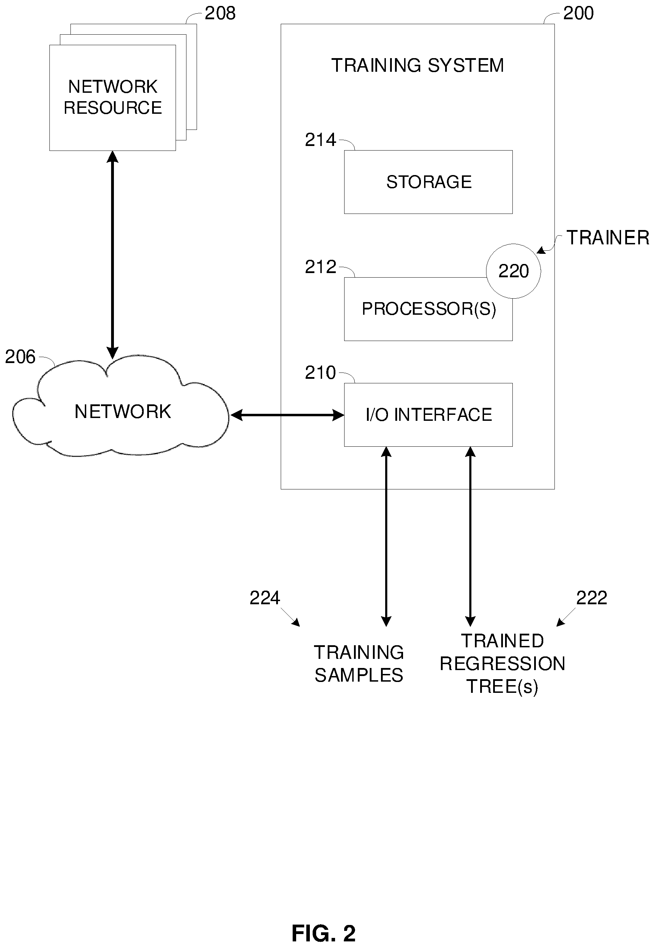

Reference is also made to FIG. 2, which is a schematic illustration of an exemplary system for training one or more regression trees to estimate a level (value) of one or more water parameters in water samples, according to some embodiments of the present invention.

An exemplary training system 200 may be configured to train one or more regression trees 222 to estimate the level of one or more of the water parameters in one or more water samples 202 of one or more bodies of water which may be natural water bodies, for example, a pool, a spring, a well, a pond, and/or the like and/or artificial man-made bodies of water, for example, a pool, a swimming pool, a water reservoir, a fountain, and/or the like collectively designated water pool herein after.

In particular, the regression tree(s) 222 may comprise one or more multivariate regression trees, for example, a PLS tree, OPLS tree, an O2PLS tree, a multivariate logarithmic regression and/or the like. Such multivariate regression trees, as known in the art, are configured to apply one or more models, for example, ML model, for example, a linear model, a neural network, a classifier, an SVM, and/or the like configured and/or trained to identify relations between multiple variants rather than simply identifying maximum variance between the response and independent variables as may be done for regular regression trees.

The training system 200 may receive a plurality of training samples 224 relating to a plurality of water samples. Each of the training samples 224 may comprise one or more physical features of a respective water sample, for example, temperature, electrical conductivity, acidity, basicity, cyanuric acid, alkalinity, hardness, and/or the like and one or more spectral features of the respective water sample, for example, light absorption (spectral response) value in one or more light spectral regions.

Each of the training samples 224 may be further associated with a value (level) of one or more of the water parameters in the respective water sample. For example, each training sample 224 may be labeled with label indicative of the chlorine concentration in the respective water sample. As such, each training sample 224 may associate the physical features and spectral features or an ORP value of a respective water sample with the level (value) of one or more of the water parameters measured for the respective water sample 202.

The training system 200, for example, a computer, a server, a computing node, a cluster of computing nodes and/or the like may include an Input/Output (I/O) interface 210, a processor(s) 212 for executing the process 100, and a storage 214 for storing data and/or computer program code (program store).

The I/O interface 210 may include one or more wired and/or wireless I/O interfaces, ports and/or interconnections, for example, a Universal Serial Bus (USB) port, a serial port, a Bluetooth (BT) interface, a Radio Frequency (RF) interface, Wireless Local Area Network (WLAN), and/or the like. Via the I/O interface 220, the training system 200 may communicate with one or more external and/or attachable devices, for example, an attachable storage media (e.g., memory stick, etc.), a nearby device (e.g., mobile device, etc.), and/or the like.

The I/O interface 210 may further include one or more wired and/or wireless network interfaces for connecting to a network 206 comprising one or more wired and/or wireless networks, for example, a Local Area Network (LAN), a WLAN (e.g. Wi-Fi), a Wide Area Network (WAN), a Metropolitan Area Network (MAN), a cellular network, the internet and/or the like. Via the I/O interface 220, the training system 200 may communicate over the network 206 with one or more remote network resources 208, for example, a server, a storage server, a cloud service, and/or the like.

Via the I/O interface 210, the training system 200 may therefore receive the training samples 224 and/or output the trained regression tree(s) 222. For example, the training system 200 may receive one or more of the training samples 224 from one or more water sampling sensors, devices, apparatuses, and/or systems (collectively designated water sampling devices) and/or the like which may connect to the network 206 and/or attach to one or more of the ports, links, and/or interfaces of the I/O interface 210. In another example, one or more of the training samples 224 captured by one or more of the water sampling devices may be stored in one or more attachable storage media devices, for example, a USB memory stick. In such case, the training system 200 may retrieve the training samples 224 from the attachable storage media devices while attached to the I/O interface 210.

Similarly, the training system 200 may output the trained regression tree(s) 222 for use by one or more estimation systems configured to use the trained regression tree(s) 222 for estimating a level and/or value of one or more of the water parameters in one or more water samples. For example, the training system 200 may transmit one or more of the trained regression tree(s) 222 to one or more remote estimation systems connected to the network 206. In another example, the training system 200 may store, transfer and/or download one or more of the trained regression tree(s) 222 to one or more attachable storage media devices attached to the I/O interface 214. The attachable storage media device(s) storing the trained regression tree(s) 222 may be then detached from the training system 200 and attached to one or more of the estimation systems which may retrieve the stored trained regression tree(s) 222.

The processor(s) 212, homogenous or heterogeneous, may include one or more processing nodes arranged for parallel processing, as clusters and/or as one or more multi core processor(s).

The storage 214 may include one or more non-transitory memory devices, either persistent non-volatile devices, for example, a ROM, a Flash array, a hard drive, an SSD, and/or the like as well as one or more volatile devices, for example, a RAM device, a cache memory and/or the like. The storage 214 may further comprise one or more local and/or remote network storage resources, for example, a storage server, a Network Attached Storage (NAS), a network drive, a cloud storage service and/or the like accessible via the network 206.

The processor(s) 212 may execute one or more software modules, for example, a process, a script, an application, an agent, a utility, a tool, an Operating System (OS), a service, a plug-in, an add-on and/or the like each comprising a plurality of program instructions stored in a non-transitory medium (program store) such as the storage 214 and executed by one or more processors such as the processor(s) 212. Optionally, the processor(s) 212 may include, utilize and/or apply one or more hardware elements available in the training system 200, for example, a circuit, a component, an Integrated Circuit (IC), an ASIC, an FPGA, a Digital Signals Processor (DSP), a Graphic Processing Unit (GPU), an Artificial Intelligence (AI) accelerator, and/or the like.

The processor(s) 212 may therefore execute one or more functional modules utilized by one or more software modules, one or more of the hardware elements and/or a combination thereof. For example, the processor(s) 212 may execute a trainer 220 configured to execute the process 100 for training one or more regression trees 222 to estimate the level and/or value of one or more of the water parameters in water samples. It should be noted that the steps of the process 100 executed by the training system 200, specifically by the trainer 220 may be executed by any of one or more processors of the processor(s) 212 such that each of the processors of the processor(s) 212 may execute the process 100 and/or part thereof.

Optionally, the training system 200, specifically, the trainer 220 may be utilized by one or more cloud computing services, platforms and/or infrastructures such as, for example, Infrastructure as a Service (IaaS), Platform as a Service (PaaS), Software as a Service (SaaS) and/or the like provided by one or more vendors, for example, Google Cloud, Microsoft Azure, Amazon Web Service (AWS) and Elastic Compute Cloud (EC2), IBM Cloud, and/or the like.

In some embodiments, the training system 200 may be integrated with one or more of the estimation systems and/or equipment, for example, a water parameters monitoring and/or control system, a cleaning system, a purifying system, and/or the like configured to apply one or more actions, operations and/or processes for controlling level and/or clean water in the water pool. In such embodiments, the chlorine estimation system may execute the trainer 220 for executing the process 100 to train one or more regression trees 222 to estimate in water samples.

For brevity the process 100 is described for training single regression tree 222. This, however, should not be construed as limiting since as may be apparent to a person skilled in the art, the process 100 may be easily expanded and/or repeated for training a plurality of regression tree to create a forest of regression trees as described herein after.

As shown at 102, the process 100 starts with the trainer 220 receiving a plurality of training samples 224 of a plurality of samples of water of one or more water pools. Each of the training samples 224 may comprise one or more physical features of a respective water sample and one or more spectral features of the respective water sample.

The physical features, for example, temperature, electrical conductivity, acidity, basicity, cyanuric acid, alkalinity, hardness, and/or the like may be extracted and/or measured through one or more tests conducted for the water samples, for example, chemical tests, electrical tests, magnetism tests, composition extraction, and/or the like.

One or more of the physical features may be measured manually for one or more of the water samples by one or more users, for example, a technician, a maintenance person, a pool owner, and/or the like using test equipment known in the art. In another example, one or more of the physical features may be measured automatically for one or more of the water samples using one or more automated systems and/or devices equipped with chemical testing equipment configured to measure one or more of the physical features. Moreover, one or more of the automated systems and/or devices, for example, a water treatment system, a water cleaning and/or purifying system, a pool robot and/or the like may have access to the water of the water pool(s) and may thus automatically take samples of the water in the water pool and measure one or more of their physical features.

The spectral features may comprise, for example, values of light absorption (response) of the water samples in one or more light spectral regions, for example, UV, fluorescence UV, UV visible, IR, NIR, fluorescence NIR, and/or the like. The spectral features may be extracted and/or measured using spectral test equipment, for example, a spectroscopic device, a spectrometer, and/or the like configured to apply one or more spectroscopic techniques as known in the art for measuring the light absorption (response) of water samples.

As described for the physical features, one or more of the spectral features may be measured manually for one or more of the water samples by one or more of the users using the spectral test equipment. In another example, one or more of the spectral features may be measured automatically for one or more of the water samples using one or more automated systems and/or devices equipped with spectral testing equipment. Moreover, one or more of the automated systems and/or devices having access to the water of the water pool(s) may automatically take samples of the pool's water and measure one or more of their spectral features.

Each of the training samples 224 may be further associated with a value of the level of one or more of the water parameters, for example, a concentration of chlorine in the respective water sample thus associating the physical features and spectral features/ORP value of each water sample with the level of the water parameter(s) measured for the respective water sample. For example, each training sample 224 may be labeled with a label indicative of the level of the water parameter(s) in the respective water sample. It should be stressed that the level of the water parameter(s) is not included in the physical features extracted and/or measured for the water samples.

As shown at 104, the trainer 220 may use the plurality of training samples 224 for training a regression tree 222, in particular, a multivariate regression tree.

The regression tree 222 may be constructed, as known in the art, of a plurality of nodes arranged in a plurality of branches each extending from a root node of the regression tree 222 to one or more leaf (end) nodes. As such each branch may include one or more nodes each split to one or more next lower level nodes until reaching leaf nodes which are not split.

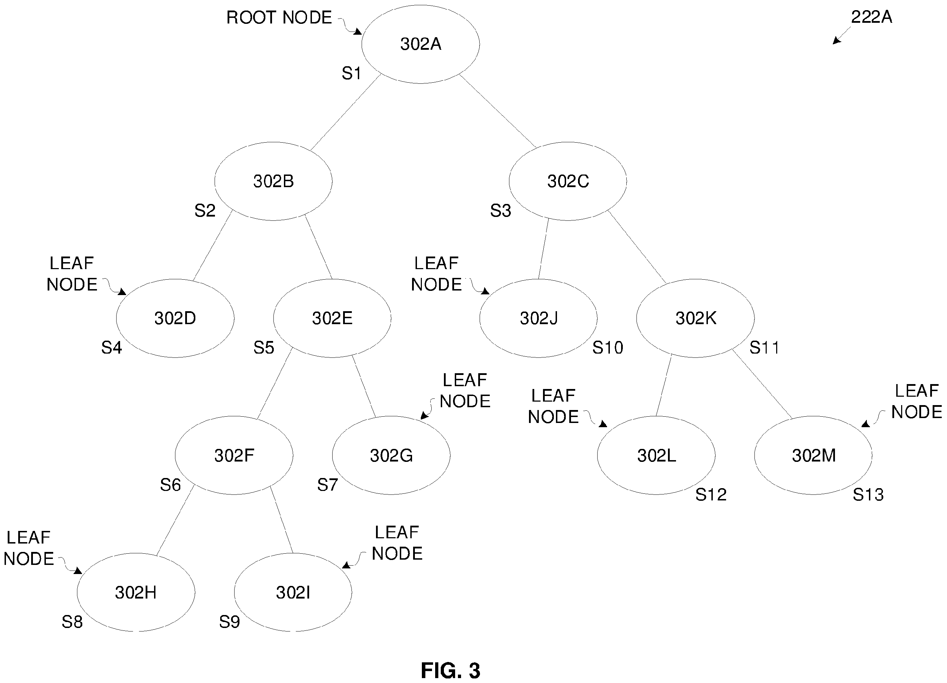

Reference is now made to FIG. 3, which is a schematic illustration of an exemplary regression tree trained for estimating a level (value) of one or more water parameters in water samples, according to some embodiments of the present invention.

An exemplary regression tree 222A may comprise a root node 302A, and two main branches each comprising a plurality of nodes 302. For example, a first branch of the regression tree 222A may comprise nodes 302B, 302D, 302E, 302F, 302G, 303H and 3021 where nodes 302D, 302G, 302H and 3021 are leaf nodes that are not further split. In another example, a second branch of the regression tree 222A may comprise nodes 302C, 302J, 302K, 302L and 302M where nodes 302J, 302L and 302M are leaf nodes that are not further split.

Reference is made once again to FIG. 1A and FIG. 1B.

The regression tree 222 may be constructed and trained in an iterative process starting from the root node and propagating towards the leaf nodes which are not split and are thus end nodes which may serve for estimating the level of the water parameter(s) in the training samples which propagated to the leaf nodes and eventually for estimating the level of the water parameter(s) in new and unseen data samples. As such, the process 100, specifically steps 106 to 126 may be repeated to create new nodes in the regression tree 222 until all branches of the regression tree 222 end with leaf nodes which are not further split.

Each node of the regression tree 222, except for the leaf nodes, may be split to two or more next lower level child nodes according to a split value selected for splitting the training (data) samples propagated to the respective node into two or more subsets of samples each propagated to one of the two or more child nodes.

The split values of the nodes are selected to achieve best prediction performance of the regression tree 222 for the training samples propagating to each of the leaf nodes. When prediction performance is not improved by splitting a certain node, the certain node may be defined as a leaf node which is not split and thus has no child nodes.

Each of the nodes of the regression tree 222 may be associated with an ML model, for example, a linear model, a neural network, a multivariate logarithmic regressiona classifier, an SVM, and/or the like which may be trained and used to estimate level of the water parameter(s) in the training samples which are propagated to the respective node. Performance of the split value selected for the parent node of their associated node may be evaluated based on a suitable norm of comparison between the predicted the level (value) of the water parameter(s) and the actual level of the water parameter(s) extracted from the labels of the training samples.

In particular, the split values evaluated and potentially selected for the nodes of the regression tree 222 may be applied to split the training samples based only on their physical features and not based on the spectral features while the ML models associated with the nodes may be trained and evaluated using only the spectral features in case of the symbolic regression tree there is no spectra input to the model, the tree splitting method is however identical to the previous example, the regression is not between the spectral and the predicted parameter, the symbolic regression tree is a group of physical/chemical parameters such as ORP, pH, water temperature etc. and environmental parameters such as temperature, UV etc.

As such, while there is a separation between the physical features and the spectral features which may significantly improve the prediction performance of the regression tree 222 and prevent overfilling, the regression tree 222 may learn the impact, relation, and/or correlation between the physical features and the spectral features.

As shown at 106, the trainer 220 may select a plurality of candidate split values for splitting the respective node (stating from the root node), which is currently processed, into two or more child nodes by splitting the training samples which propagated to the respective node into two or more subsets each propagated into one of the two or more child nodes.

In particular, the trainer 220 may select the plurality of candidate split values based only on one or more of the physical features of the training samples which are propagated to the respective node, for example, temperature, electrical conductivity, acidity, basicity, and/or the like. For example, the trainer 220 may select a first electrical conductivity value as a first candidate split value, a second electrical conductivity value as a second candidate split value, a first acidity value as a third candidate split value, a second acidity value as a fourth candidate split value, a third acidity value as a fifth candidate split value, a first alkalinity value as a sixth candidate split value, a second alkalinity value as a seventh candidate split value, a certain temperature value as an eighth candidate split value, and/or the like.

The trainer 220 may further select one or more candidate split values based on multiple physical features. For example, the trainer 220 may select a third electrical conductivity value and a first temperature value as a ninth candidate split value, a fourth electrical conductivity value and a fourth acidity value as a tenth candidate split value, a third alkalinity value, a second temperature value and a fifth acidity value as an eleventh candidate split value, and so on.

One or more of the plurality of physical features may be expressed in discrete values, i.e., nominal values. For example, acidity may be expressed in 16 discrete pH values in a range of 6.8 to 8.4. However, the physical features may be typically expressed in a continuous range. For example, temperature may be expressed in one or more scales and/or ranges, for example, Celsius, Fahrenheit, Kelvin, and/or the like. In another example, acidity and/or basicity may be expressed in one or more scales and/or ranges, for example, pH, and/or the like. In another example, electrical conductivity may be expressed in one or more scales and/or ranges, conductance, resistance, current flow, and/or the like.

Optionally, the trainer 220 may further select one or more candidate split values based a plurality of quantiles segmenting the continuous range and/or scale of one or more of the physical features expressed a continuous range. For example, rather than setting one or more candidate split values to define a singular temperature value, the temperature range of the water samples may be segmented to several quantiles of essentially equal size, and the candidate split values may be selected to define one or more of the quantiles.

Segmenting the continuous ranges of the physical features to quantiles may serve to limit the size of the regression tree 222 and the number its nodes thus preventing an excessive number of ML models. As such, the computing resources, for example, processing resources, storage resources, network resources, processing time and/or the like required for training the ML models associated with the nodes may be controlled, reduced and/or fitted according to resources availability.

It should be stressed that as the regression tree 222 expands, the training samples may disperse across the multiple branches and nodes of the regression tree 222 such that, except for the root node, only part of the training samples may reach each of the lower levels nodes with the number of training samples arriving at each node reducing as they propagate down the regression tree 222 towards the leaf nodes.

For example, as seen in FIG. 3, a set of training samples S1 used to train the regression tree 222A may be injected to the root node 302A. The set S1 may be split to two subsets S2 and S3 where S2 is propagated to node 302B and S3 is propagated to node 302C. Moving further along the first branch of the regression tree 222A, the subset S2 may be split into two subsets S4 and S5 propagated to nodes 302D and 302E respectively. The subset S5 is further split into two subsets S6 and S7 propagated to nodes 302F and 302G respectively and the subset S6 is split again into two subsets S8 and S9 propagated to nodes 302H and 302I respectively. In the second branch of the of the regression tree 222A, the subset S3 may be split into two subsets S10 and S11 propagated to nodes 302J and 302K respectively and the subset S11 is split again into two subsets S12 and S13 propagated to nodes 302L and 302M respectively. This means that the subsets of training samples S4, S7, S8, S9, S10, S12 and S13, combined , form the set of training samples S1 injected to the root node 302 where only part of the set S1 reaches each of the leaf nodes.

Moreover, in order to ensure robustness, reliability, and/or accuracy of the regression tree 222, the trainer 220 may be configured to ensure that a minimal predefined number of samples is propagated to each node of the regression tree 222. The trainer 220 may therefore select and/or adjust one or more of the candidate split values of each node of the regression tree 222 to ensure that each of the subsets propagated to the child nodes of the respective node comprise at least the minimal predefined number of training samples.

Furthermore, since the subsets of training samples are split over and over as the training samples propagate down the regression tree 222, the number of training samples included in the subsets is constantly reduced. The minimal number of of training samples predefined for the subsets may be therefore adjusted according to the level of the nodes in the regression tree 222.

As shown at 108, the trainer 220 may propagate the training samples which propagated to the respective (currently processed) node to two child nodes of the respective node according to one of the selected candidate split values. In particular, the trainer 220 may split the training samples which propagated to the respective node into two subsets and propagate a first subset to a first child node and a second subset to a second child node.

To this end, the trainer 220 may compare between the relevant (applicable) physical value(s) of each of the propagated training samples and the selected candidate split value and propagate the training samples accordingly. For example, assuming a certain split value defines a certain acidity value, the trainer 220 may propagate all training samples having acidity lower than the certain acidity value to a first one of the two child nodes. Complementary, the trainer 220 may propagate all training samples having acidity higher and/or equal to the certain acidity value to a second one of the two child nodes. In another example, assuming a certain split value defines a certain electrical conductivity value and a certain temperature value. In such case, the trainer 220 may propagate all training samples having electrical conductivity higher than the certain electrical conductivity value and temperature lower than the certain temperature value to the first child node and all training samples having electrical conductivity lower and/or equal than the certain electrical conductivity value and temperature higher than the certain temperature value to the second child node.

As shown at 110, the trainer 220 may train the two ML models associated with the two child nodes to estimate the level of the water parameter(s) in each of the training samples of the subset propagated to the child nodes. This means, that the trainer 220 may use the first subset of training samples to train the ML model associated with the first child node and the second subset of training samples to train the ML model associated with the second child node.

Optionally, the trainer 220 may train the two ML models using only the spectral features of the training samples of the propagated subsets, for example, light absorption (response) values of the water samples in one or more light spectral regions while discarding their physical features.

The trainer 220 may apply one or more training techniques and/or paradigms, for example, supervised training, semi-supervised training, unsupervised training and/or a combination thereof to train the ML models. For example, the trainer 220 may train one or more of the ML models associated with the child nodes in supervised learning using the water parameter(s) values measured for the water samples which are associated with at least some of the (labeled) training samples. In another example, the trainer 220 may apply unsupervised learning and train one or more of the ML models to cluster unlabeled training samples in a plurality of clusters and estimate their water parameter(s) levels accordingly. In another example, the trainer 220 may apply semi-supervise learning to train one or more of the ML models using a combination of labeled and unlabeled training samples.

As shown at 112, the trainer 220 may evaluate performance of each of the two ML models associated with the child nodes in its prediction of the level of one or more of the water parameters in the training samples of the subset which propagated to the respective associated child node.

In particular, the trainer 220 may apply the trained ML models associated with the two child nodes to estimate (predict) a value of water parameter(s) in the training samples of their respective subsets and compare the estimated water parameter(s) values with the actual water parameter(s) values associated with these training samples.

For example, the trainer 220 may apply one or more optimization functions and/or algorithms to minimize loss of the trained ML models associated with each of two or more child nodes which is applied to estimate the level of the water parameter(s) in the training samples of the respective subset which propagated to the respective child node.

Moreover, in order to prevent fitting of the ML models to their training data, the trainer 220 may divide the training samples of each subset propagated to each child node to two non-overlapping groups, i.e., the two groups comprise different training samples. A first group of the two groups may comprise training samples serving as training samples and a second group of training samples serving as validation samples.

The trainer 220 may thus use the training samples of the first group to train the ML model associated with the respective node as described in step 110. The trainer 220 may then use the training samples of the second group to test the ML model associated with the respective node and evaluate its prediction performance. Typically, the first group may be larger than the second group and may therefore comprise more training samples than the second group. For example, the first group may comprise 80% of the training samples included in the respective subset while the second group may comprise 20% of the training samples of the respective subset.

Optionally, the trainer 220 may divide the training samples of each subset propagated to each child node to three groups, a first group used to train the ML model, a second group used to test the ML model and a third group used to evaluate the ML model. For example, the first group may comprise 80% of the training samples included in the respective subset, the second group may comprise 10% of the training samples of the respective subset and the third group may comprise the remaining 10% of the training samples of the respective subset.

As shown at 112, which is a conditional step, the trainer 220 may check whether the performance of the ML models associated with the child nodes, for example, accuracy, consistency, reliability, and/or the like, is improved compared to other candidate split values previously selected for the respective (currently processed) node.

Each split value may yield different subsets of values propagated to the child nodes such that the ML models associated with the child nodes are trained and evaluated using different sets of training samples. The performance of the ML models may therefore vary depending on the split values applied to the parent node of their associated nodes.

In case the performance of the ML models evaluated by the trainer 220 by minimizing the loss across the prediction of the level of the water parameter(s), for example, (free) chlorine concentration in the training samples of the subsets, is improved compared to the other candidate split values, the process 100 may branch to 116. Otherwise the trainer 220 may discard the currently evaluated candidate split value and branch to 118.

As shown at 116, the trainer 220 may set (and save) the currently selected candidate split value as the optimal split value since the performance of the ML models trained and tested using the training samples subsets defined by the current candidate split value exceeds the performance of the ML models trained and tested according to the previous candidate split values.

As shown at 118, which is another conditional step, in case there are additional candidate split values which are not evaluated yet, the trainer 220 may branch back to step 108 and repeat the evaluation using another candidate split value, otherwise the process 220 may continue to 120.

As shown at 120, which is also a conditional step, the trainer 220 may check whether the performance of the ML models with the child nodes in predicting the level of the water parameter(s) in the training samples is improved compared to the performance of the ML model associated with the parent node, i.e., the currently processed node. In case the performance of the child nodes is improved compared to the parent node, thus justifying the split of the current node, the process 100 may branch back to 122, otherwise the process may branch to 124.

As shown at 122, since the performance of the child nodes is improved compared to their parent node, the trainer 220 may split the current node to two child nodes according to the optimal candidate split value which yielded best performance among all candidate split values.

Since the currently processed node is split, there must be at least two child nodes that need to be further processed and evaluated and the process may branch back to 106 to start processing another node of the regression tree 222.

As shown at 124, since the performance of the child nodes does not improve over that of the currently processed node, the trainer 220 may not split the current node and may rather define it as a leaf node. In particular, the loss computed by the trainer 220 for the ML models associated with the child nodes of the current node exceeds the loss computed for the ML model associated with the parent node, i.e., the currently processed node, therefore the parent node (current node) is not split and is defined as a leaf node.

Optionally, one or more leaf nodes may be pruned in case the number of samples which propagate to these leaf nodes does not exceed a predefined minimum number.

An exemplary algorithm *PLSNode*() which may be applied for constructing a regression tree 222 as described in steps 104 to 126 is presented in pseudo-code excerpt 1 below.

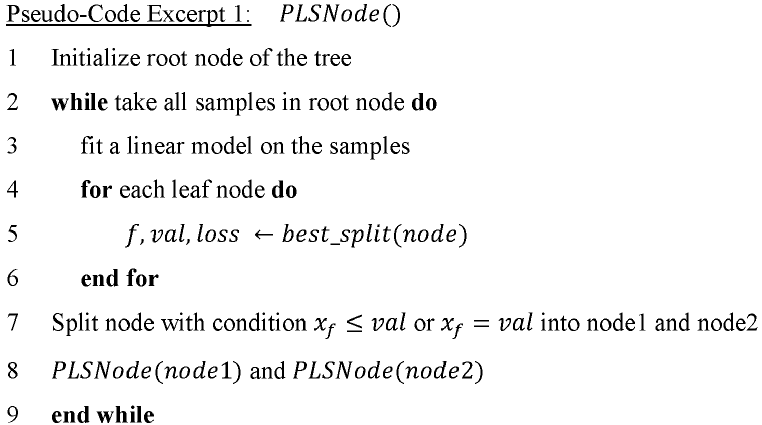

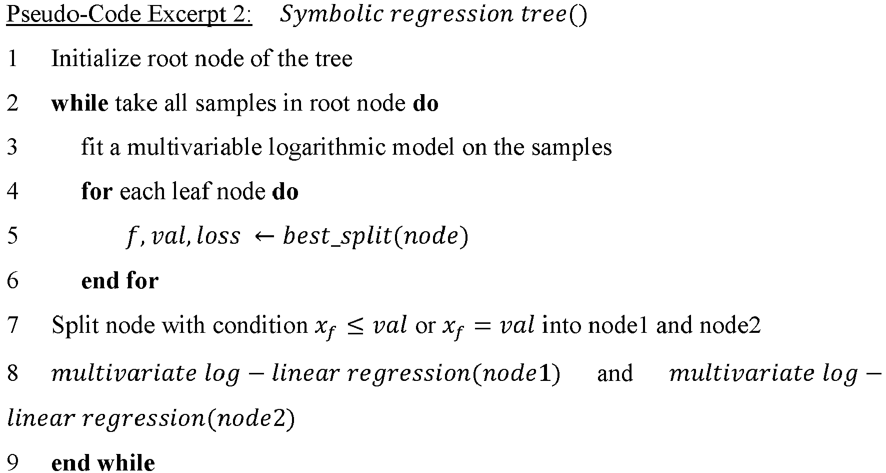

As seen, both the algorithm *PLSNode*() and *Symbolic regression tree*()are iterative and recursive algorithms which traverse nodes of the regression tree 222 starting from the root node until reaching the leaf nodes. The algorithm *PLSNode*() or the SRT() may employ an exemplary split value selection function *best_split*(*node*) presented in pseudo-code excerpt 3 below. The function *best_split*(*node*) executed for each node *n* may traverse each of the physical features f of the training samples to select a plurality of candidate split values (split points) and evaluate the performance *ΔL*(*n, f, val*) of the ML models associated with its child nodes compared to the performance *L_{Parent}* of the parent node to determine whether to further split the node n or define it as a leaf node.

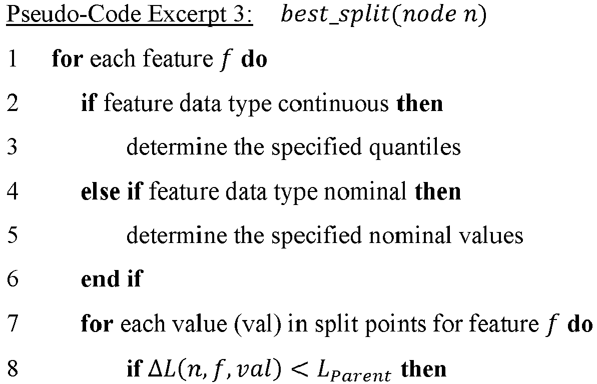

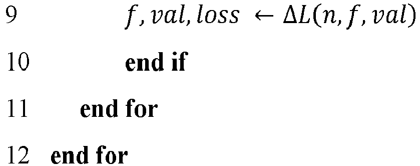

As shown at 126, the trainer 220 may check whether there are non-leaf nodes in the regression tree 222 which are not are not processed yet to determine whether they are leaf nodes or they should be further split. In case there are one or more unprocessed nodes, the process may branch to 106 to start processing another node of the regression tree 222. Otherwise the process 100 may branch to 128.

As shown at 128, the training and construction of the regression tree 222 is complete as all its nodes are processed.

Optionally, the trainer 220 may repeat the process 100 to create and train one or more additional regression trees such as the regression tree 222 to create and form a forest of regression trees comprising a plurality of trained regression trees 222. The trainer 220 may apply one or more methods for constructing and training the additional regression tree(s) 222.

For example, the trainer 220 may train one or more additional regression trees 222 having different association of their nodes with respective ML models compared to the association of the first regression tree 222. For example, the ML model associated with one or more nodes of a first regression tree 222 may be configured differently and/or employ a different architecture compared to corresponding nodes in a second regression tree 222.

In another example, the trainer 220 may train the additional regression tree(s) 222 using one or more other sets of training samples different from the training samples used to train the first regression tree 222. In another example, the trainer 220 may split the set of training samples to multiple sets and use each of the sets to train a respective regression tree 222. In another example, the trainer 220 may use the same set of training samples to train multiple regression trees 222 having unique split value configurations for at least some of their nodes. For example, a first regression tree 222 may be constructed and trained using split values based on a single physical feature while a second regression tree 222 may be constructed and trained using combined split values defining multiple physical features for at least some of its nodes.

As shown at 130, the trainer 220 may output the trained regression tree(s) 222 which may be used to estimate and/or predict the level of the water parameter(s), for example, the concentration of (free) chlorine in one or more water samples, specifically water samples which were not previously processed by the regression tree(s) 222, i.e., not used for training the regression tree(s) 222.

The trainer 220 may output the trained regression tree(s) 222 in one or more ways. For example, as described herein before, the trainer 220 may transmit the trained regression tree(s) 222 or appropriate subset thereof, e.g., corresponding to "physical" properties now existing in the pool via the network 206 to one or more remote estimation systems, apparatuses, and/or equipment which may use the trained regression tree(s) 222 to monitor one or more of the water parameters in a water pool and optionally initiate and/or apply one or more actions, operations and/or processes for controlling and/or cleaning the water in the water pool accordingly. In another example, the trainer 220 may store, transfer and/or download the trained regression tree(s) 222 to one or more attachable storage media devices attached to the I/O interface 214 which may be detached and connected to one or more of the chlorine estimation systems which may retrieve the stored trained regression tree(s) 222.

In another example, the trainer 220 and the training system 200 may be integrated with one or more such estimation systems, apparatuses, and/or equipment which may use the trained regression tree(s) 222. In such case the trainer 220 may locally store (save) the trained regression tree(s) 222, for example, in the storage 214.

As seen, there may be some overlap between the measured physical features and the predicted water parameters. This is because as described herein before, in some embodiments one or more of the water parameters are predicted while in other embodiments the same water parameter(s) may be measured and used for predicting one or more of the other water parameters. For example, in some embodiments, an alkalinity (value) of the water may be predicted based on one or more physical features measured in one or more water samples, for example, acidity, temperature, electrical conductivity, and/or the like. However, in other embodiments, the alkalinity (value) of the water may be measured and used to predict another water parameter, for example, chlorine concentration.

The training samples used to train the regression tree(s) may be therefore configured, selected, and/or constructed to comprise one or more of the water parameters as physical features which are measured in the training water samples.

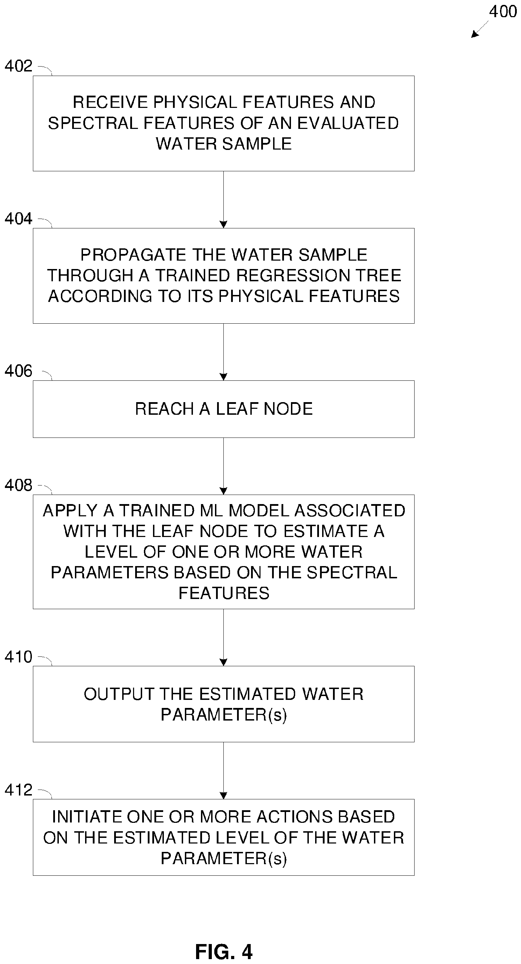

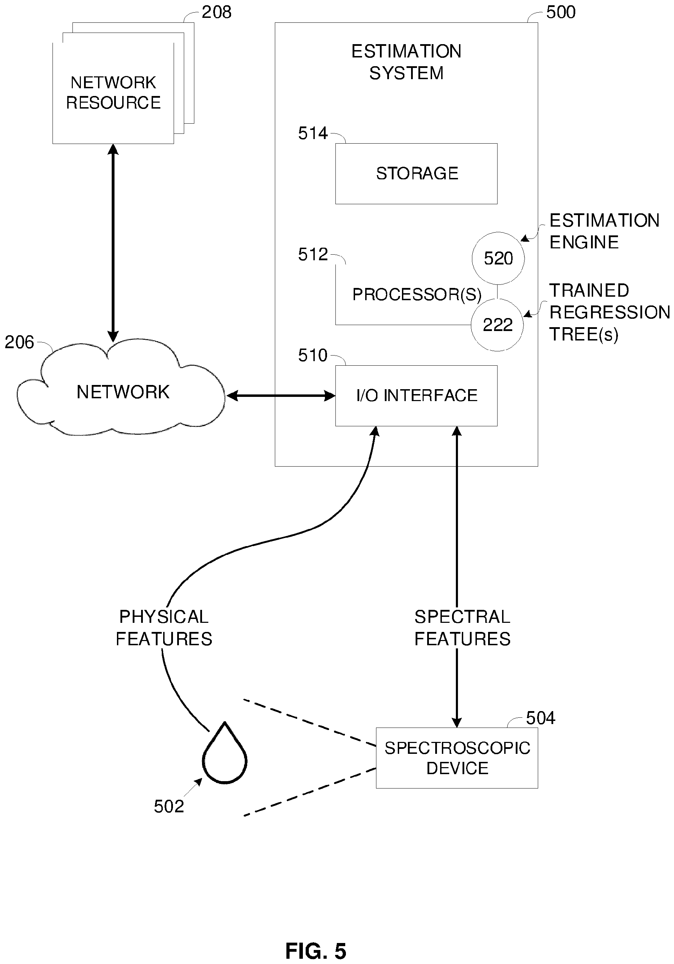

Reference is now made to FIG. 4, which is a flowchart of an exemplary process of estimating a level (value) of one or more water parameters in water samples using one or more trained regression trees, according to some embodiments of the present invention. Reference is also made to FIG. 5, which is a schematic illustration of an exemplary system for estimating a level (value) of one or more water parameters in water samples using one or more trained regression trees, according to some embodiments of the present invention.

An exemplary process 400 for estimating a level of one or more of the water parameters in water samples may be executed by an exemplary estimation system 500 using a trained regression tree such as the regression tree 222, in particular a trained multivariate regression tree.

The estimation system 500, for example, a computer, a server, a computing node, a cluster of computing nodes and/or the like may include an I/O interface 510 such as the I/O interface 210, a processor(s) 512 such as the processor(s) 212 for executing the process 400, and a storage 514 such as the storage 214 for storing data and/or computer program code (program store).

Via the I/O interface 510, the estimation system 500 may receive spectral features and physical features of one or more water samples 502. For example, the estimation system 500 may communicate with one or more spectroscopic devices 504 configured to capture, measure, and/or otherwise extract one or more spectral features of the water sample(s) 502, for example, a light absorption in one or more spectral regions such as, for example, UV, fluorescence UV, UV visible, IR, NIR, fluorescence NIR, and/or the like. In another example, the estimation system 500 may communicate with one or more remote resources 208 via a network such as the network 206 to receive one or more physical features of the water sample(s) 502 captured, measured, and/or otherwise extracted manually and/or automatically as described herein before.

Via the I/O interface 510, the estimation system 500 may also receive one or more regression trees 222 trained to estimate the level of one or more of the water parameters in water samples 502. For example, the estimation system 500 may communicate, via the network 206, with one or more remote systems, for example a training system such as the training system 200 to receive one or more trained regression trees 222.

The processor(s) 512 may execute one or more functional modules utilized by one or more software modules, one or more hardware elements available in the estimation system 500, and/or a combination thereof. For example, the processor(s) 512 may execute an estimation engine 520 configured to execute the process 400 for estimating the level of the water parameter(s) in water samples using the trained regression tree 222. It should be noted that, as stated for the processor(s) 212, the steps of the process 400 executed by the estimation system 500, specifically by the estimation engine 520 may be executed by any of one or more processors of the processor(s) 512 such that each of the processors of the processor(s) 512 may execute the process 400 and/or part thereof.

Optionally, the estimation system 500, specifically, the estimation engine 520 may be utilized by one or more cloud computing services, platforms and/or infrastructures such as, for example, IaaS, PaaS, SaaS and/or the like provided by one or more vendors, for example, Google Cloud, Microsoft Azure, AWS and EC2, IBM Cloud, and/or the like.

Optionally, the estimation system 500 may be integrated in one or more water treatment systems, for example, automated systems and/or devices, for example, a water treatment system, a water cleaning and/or purifying system, a pool robot and/or the like such that the estimation engine 520 may be executed by the water treatment system(s).

For brevity the process 400 is described for estimating the level of the water parameter(s) in a single water sample 502 using a single trained regression tree 222. This, however, should not be construed as limiting since as may be apparent to a person skilled in the art, the process 400 may be easily expanded and/or repeated for estimating the level of the water parameter(s) in a plurality of water samples 502 and/or for using a forest comprising a plurality of trained regression trees 222.

As shown at 402, the process 400 starts with the estimation engine 520 receiving the spectral features and physical features a water sample 502.

As shown at 404, the estimation engine 520 may propagate the water sample 502 through the trained regression tree 222 based on the physical features of the water sample 502.

As shown at 406, the estimation engine 520 may propagate the water sample 502 through the trained regression tree 222 until it reaches one of the leaf nodes of the regression tree 222 which is the end node for the evaluated water sample 502.

A shown at 408, the estimation engine 520 may identify the trained ML model associated with the end leaf node, to which the water sample 502 propagated, and apply the ML model to the spectral features of the water sample 502 to compute an estimated the level and/or value of one or more of the water parameters for the water sample 502.

A shown at 410, the estimation engine 520 may output the chlorine concentration value estimated for the water sample 502. For example, the estimation engine 520 may transmit the estimated water parameter(s) value over the network 206 to one or more remote resources 208, for example, an automated system, apparatus and/or equipment, for example, a water monitoring and/or control system, a cleaning system, a purifying system, and/or the like configured to apply one or more actions, operations and/or processes for controlling, treating, and/or cleaning the water in the water pool from which the water sample 502 is taken.

As shown at 412, the estimation engine 520, in particular when integrated in a water treatment system, may optionally initiate one or more actions, operations and/or processes for controlling the water in the water pool from which the water sample 502 is taken based on the estimated level of one or more of the water parameters. For example, assuming the estimated water parameter is chlorine concertation. In case the value of chlorine concertation estimated by the trained regression tree 222 is below a certain first threshold, the estimation engine 520 may initiate one or more actions to administer chlorine into the water pool until the chlorine concertation estimated for the water pool reaches and/or exceeds the first threshold. In another example, in case the chlorine concertation value estimated by the trained regression tree 222 is below a certain second threshold which is significantly lower than the first threshold, the estimation engine 520 may initiate further action(s) to clean the water in the water pool beyond just administering chlorine into it. In another example, in case the chlorine concertation value estimated by the trained regression tree 222 for the water sample 502 is significantly above the first threshold, the estimation engine 520 may initiate one or more actions to reduce chlorine level in the water pool, for example, circulate the water in the water pool, replace some water, and/or the like.

As described herein before, one or more of the water parameters may be predicted using the regression tree(s) in some embodiments, while in other embodiments, the same water parameters may be measured and used to predict one or more other water parameters. For example, in some embodiments, the concentration level (value) of oxygen dissolved in the water may be predicted based on one or more of the physical features measured in the water, for example, acidity, alkalinity, hardness, and/or the like, while in other embodiments, the dissolved oxygen alkalinity concentration may be measured and used to predict another water parameter, for example, chlorine concentration.

The descriptions of the various embodiments of the present invention have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

It is expected that during the life of a patent maturing from this application many relevant systems, methods and computer programs will be developed and the scope of the terms spectroscopic device, regression tree, multivariate regression tree, and ML model are intended to include all such new technologies a priori.

As used herein the term "about" refers to ± 10 %.

The terms "comprises", "comprising", "includes", "including", "having" and their conjugates mean "including but not limited to". This term encompasses the terms "consisting of" and "consisting essentially of".

The phrase "consisting essentially of" means that the composition or method may include additional ingredients and/or steps, but only if the additional ingredients and/or steps do not materially alter the basic and novel characteristics of the claimed composition or method.

As used herein, the singular form "a", "an" and "the" include plural references unless the context clearly dictates otherwise. For example, the term "a compound" or "at least one compound" may include a plurality of compounds, including mixtures thereof.

The word "exemplary" is used herein to mean "serving as an example, an instance or an illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals there between.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

It is the intent of the applicant(s) that all publications, patents and patent applications referred to in this specification are to be incorporated in their entirety by reference into the specification, as if each individual publication, patent or patent application was specifically and individually noted when referenced that it is to be incorporated herein by reference. In addition, citation or identification of any reference in this application shall not be construed as an admission that such reference is available as prior art to the present invention. To the extent that section headings are used, they should not be construed as necessarily limiting. In addition, any priority document(s) of this application is/are hereby incorporated herein by reference in its/their entirety.

## Claims

1. A method of creating machine learning (ML) based regression trees trained for estimating a level of at least one water parameter, comprising:
using at least one processor for:
using a plurality of training samples relating to a plurality of water samples to create a regression tree comprising a plurality of nodes arranged in a plurality of branches extending from a root node to a plurality of leaf nodes by splitting each of the plurality of nodes into at least two respective child nodes by:
splitting a set of the plurality of training samples which propagated to the respective node into at least two subsets based on each of a plurality of candidate split values applied for each of at least one of a plurality of physical features of the propagated samples,
for each of the candidate splits, training two ML models each associated with one of the child nodes, each ML model is trained using a plurality of spectral features of the propagated samples of a respective one of the two subsets and its loss is computed, and
selecting an optimal split value from of the plurality of candidate split values by minimizing loss of each of the trained ML models applied to estimate a level of at least one water parameter in the propagated samples of the respective subset or not splitting if neither split improves the loss; and
outputting the regression tree for estimating the level of the at least one water parameter in at least one new water sample.

2. The method of claim 1, wherein the at least one water parameter is a member of a group consisting of: a chlorine concentration value, a dissolved oxygen concentration value, an alkalinity value, and an acidity value.

3. The method of any of the previous claims, wherein each of the plurality of training samples is associated with a respective value of a level of the at least one water parameter.

4. The method of any of the previous claims, wherein the loss is computed based on the level of the at least one water parameter of the plurality of training samples.

5. The method of any of the previous claims, wherein the plurality of candidate split values are selected to ensure a minimal predefined number of samples is propagated to each of the child nodes.

6. The method of any of the previous claims, wherein in case a minimized loss computed for a certain node does not exceed the loss computed for its parent node, the parent node is not split and is defined as a leaf node.

7. The method of any of the previous claims, wherein each of the ML models associated with one of the at least two child nodes is trained using a first group of training samples selected from one of the subsets which is propagated to the respective child node and tested using a second group of validation samples of the respective subset such that the first group is not overlapping with the second group;, wherein the first group is optionally larger than the second group.

8. The method of any of the previous claims, wherein the ML models are linear ML models.

9. The method of any of the previous claims, wherein the plurality of physical features are members of group consisting of: temperature, electrical conductivity, acidity, basicity, cyanuric acid, alkalinity, and hardness.

10. The method of any of the previous claims, wherein the plurality of spectral features comprise light absorption values in at least one of a plurality of light spectral regions and/or at least one of the plurality of physical features is expressed in discrete values.

11. The method of any of the previous claims, wherein at least one of the plurality of physical features is expressed in a continuous range; wherein the plurality of candidate split values are optionally selected based on a plurality of quantiles segmenting the continuous range of the at least one physical feature.

12. The method of any of the previous claims, wherein the plurality of samples are samples of water of at least one pool.

13. The method of any of the previous claims, further comprising creating a forest of regression trees comprising a plurality of regression trees, each of the plurality of regression trees is created based on a different association of its plurality of nodes with respective ML models.

14. A system for creating machine learning (ML) based regression trees trained for estimating a level of at least one water parameter, comprising:
at least one processor configured to execute a code, the code comprising:
code instructions to use a plurality of training samples relating to a plurality of water samples to create a regression tree comprising a plurality of nodes arranged in a plurality of branches extending from a root node to a plurality of leaf nodes by splitting each of the plurality of nodes into at least two respective child nodes by:
splitting a set of the plurality of training samples which propagated to the respective node into at least two subsets based on each of a plurality of candidate split values applied for each of at least one of a plurality of physical features of a plurality of the training samples,
training two ML models each associated with one of the child nodes, each ML mode is trained using a plurality of spectral features of the propagated samples of a respective one of the two subsets, and
selecting an optimal split value from of the plurality of candidate split values by minimizing loss of each of the trained ML models applied to estimate a level of at least one water parameter in the propagated samples of the respective subset; and
code instructions to output the regression tree for estimating the level of the at least one water parameter in at least one new water sample.

15. A method of estimating a level of at least one water parameter using trained machine learning (ML) based regression trees, comprising:
using at least one processor for:
receiving a plurality of physical features and a plurality of spectral features relating to at least one sample of water;
propagating the at least one sample of water through a regression tree comprising a plurality of nodes arranged in a plurality of branches propagating from a root node to a plurality of leaf nodes, each of the plurality of nodes is associated with an ML model trained to estimate a level of at least one water parameter in water samples;
identifying a certain leaf node of the plurality of leaf nodes to which the at least one water sample propagated based on the plurality of physical features;
estimating a level of the at least one water parameter in the at least one water sample by applying the ML model associated with the certain leaf node to the plurality of spectral features; and
initiating at least one action based on the estimated level of the at least one water parameter; optionally further comprising estimating the level of the at least one water parameter in the at least one water sample using a forest of regression trees comprising a plurality of regression trees, each of the plurality of regression trees is configured with different association of its plurality of nodes with respective ML models.
